# EUROPEAN PATENT APPLICATION

(11) **EP 1 236 744 A2**
(43) Date of publication of application: **04.09.2002**
(21) Application number: 02004715.5
(22) Date of filing: 01.03.2002
(51) Int. Cl.: C08F 26/02, C08F 20/34, A61K 7/48, A61K 7/06

(54) **Guanidin-group containing polymer, cosmetic employing same and method of preparing same**

(30) Priority: 01.03.2001 JP 2001056311
(71) Applicant: MITSUBISHI CHEMICAL CORPORATION, Chiyoda-ku, Tokyo 100-0005 (JP)
(72) Inventor: Hiwatashi, Tomoaki, Mitsubishi Chemical Corp., Yokkaichi-shi, Mie 510-8530 (JP); Oouchi, Shinsuke, Mitsubishi Chemical Corporation, Yokkaichi-shi, Mie 510-8530 (JP); Kitani, Yasuo, Mitsubishi Chemical Corporation, Yokkaichi-shi, Mie 510-8530 (JP)
(74) Representative: Werner, Hans-Karsten, Dr.Dipl.-Chem.

(57) **Abstract**

A cosmetic-use polymer having a weight average molecular weight of 5.0·10³-1.0·10⁷ and comprising a repeating unit having at least the guanidino group denoted by general formula (I) below 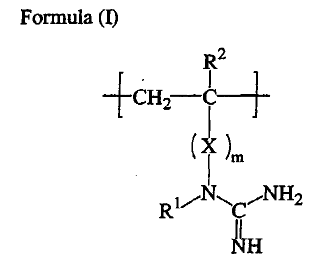 (where, in the formula, R¹ denotes a hydrogen atom or hydrocarbon group, R² denotes a hydrogen atom or methyl group, X denotes a bivalent connecting group, m denotes 0 or 1, and the guanidino group in the formula is optionally a salt to which an acid has been added) was disclosed.

## Description

### Technical Field

The present invention relates to a new cosmetic-use polymer, a cosmetic employing the same, and a method of preparing the same.

### Related Art

Cosmetics employing polymers having film-forming properties are conventionally known as cosmetics for use on the skin, nails, and hair. For example, cosmetics are known that serve the purpose of improving the adhesion of cosmetics employed on the skin and nails, protecting the skin and nails, or imparting color or some other form of decoration to the skin and nails. Hair cosmetics are known that serve the purpose of imparting setting strength and flexibility to the hair. Various anionic, cationic, and amphoteric polymers are employed in these cosmetics to impart film forming properties.

For example, properties such as permitting ready removal by washing and ready dilution during preparation are required of the polymers employed in hair cosmetics. Such water-soluble polymers include nonionic, anionic, and cationic polymers. Nonionic polymers that are employed in hair cosmetics include polyvinylmethylether and polyvinylpyrrolidone. However, hair cosmetics comprising polyvinylpyrrolidone as a component have the drawback of properties varying widely with temperature and humidity. For example, under conditions of low humidity, films of the above-mentioned polymers formed on the hair are quite hard and tend to flake. By contrast, under conditions of high temperature and high humidity, these films become extremely soft, blocking occurs, and individual hairs clump together, tending to make combing and brushing difficult. When the above-mentioned polyvinylpyrrolidone is employed, the effects of humidity become even more prominent.

Examples of anionic polymers include copolymers obtained by copolymerization of a vinyl carboxylic acid such as acrylic acid or methacrylic acid with styrene and/or an acrylic alkyl ester. In contrast to the above-mentioned nonionic polymers, the copolymer obtained tends not to be affected by temperature and humidity. However, since it is anionic in the same manner as the hair, there is a drawback in the form of low affinity with hair. Hard films are generally formed, which is desirable for achieving hairdressing effects, but there is also a drawback in that flaking tends to occur. Since the copolymer is anionic, the addition of cationic substances is limited, and there is a risk that rinses (cationic substances) will cause solidification when the hair is washed. To alleviate the drawbacks of hair cosmetics comprising nonionic polymers or anionic polymers, the use of an amphoteric polymer in the form of a copolymer employing a carboxybetaine portion as a hydrophilic group has been proposed. These amphoteric polymers are known to be good polymers for hairstyling from the perspectives of compatibility with hair and setting strength. Hairstyling-use polymers (JP-A-51-9732 and JP-A-55-104209; the term "JP-a" as used herein means an "unexamined published Japanese patent application) comprising amphoteric polymers obtained by converting with a halogenated acetate of a terpolymer comprising (a) 20-60 weight percent of a tertiary amine-comprising (meth)acrylic acid (the term "(meth)acrylic acid" will hereinafter embrace both acrylic acid and methacrylic acid) ester unsaturated monomer, and 30-70 weight percent of a mixture of (b) a (meth)acrylic acid alkyl ester comprising an alkyl group with 1-4 carbon atoms and (c) a (meth)acrylic acid alkyl ester comprising an alkyl group with 12-18 carbon atoms have been developed.

Although cationic polymers have good affinity with hair, they have the drawback of being affected by humidity as is the case with nonionic polymers. Further, since they are cationic polymers, the addition of anionic substances is limited, and there is a risk of solidification by shampoos (anionic) during hair washing. Generally, there is a problem in that the setting strength drops as the water solubility of the polymer increases, making it difficult to achieve both ease of washing and setting strength. No conventional hair cosmetic employing polymers has been provided that fully satisfies all of the properties desired in a hair cosmetic. Specifically, no hair cosmetic has yet been provided that simultaneously imparts to the hair adequate setting strength and a flexible feeling during use while affording ease of removal by washing the hair.

Additionally, various anionic, cationic, and amphoteric polymers are also employed in skin and nail cosmetics. However, films of conventional polymers are often lacking in the feelings they impart during use, such as pricking feelings, unnatural feelings, or tacky feelings imparted to the skin. Further, film formation is sometimes inadequate and the desired effects are sometimes not achieved. With nail cosmetics, there are problems in that some polymers have poor adhesion to nails.

The present invention, devised in light of the various above-described problems, has for its object to provide a cosmetic polymer, and method of preparing the same, having good adhesion and film forming properties on skin, nails, and hair, good water solubility, and no feeling of tackiness or unnaturalness due to film formation. A further object of the present invention is to provide a cosmetic with good adhesion and film forming properties on skin and nails that imparts a pleasant feeling with use and does not impart a feeling of tackiness or unnaturalness due to film formation. A still further object of the present invention is to provide a hair cosmetic with good adhesion and film forming properties on hair that imparts a pleasant feeling when used and does not impart a feeling of tackiness or unnaturalness due to film formation. And a still further object of the present invention is to provide a hair cosmetic that washes out readily and imparts good setting strength and a flexible style to the hair.

### Summary of the Invention

The present inventors conducted extensive research, resulting in the discovery that the above-stated problems could be solved by means of a polymer comprising a guanidino group having a specific structure; the present invention was devised on that basis.

The foregoing objects are accomplished by the invention to provide a cosmetic-use polymer having a weight average molecular weight of 5.0·10³-1.0·10⁷ and comprising a repeating unit having at least the guanidino group denoted by general formula (I) below 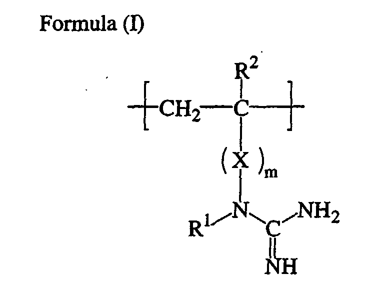 (where, in the formula, R¹ denotes a hydrogen atom or hydrocarbon group, R² denotes a hydrogen atom or methyl group, X denotes a bivalent connecting group, m denotes 0 or 1, and the guanidino group in the formula is optionally a salt to which an acid has been added).

In the formula (I), X preferably denotes a connecting group comprising one or more members selected from the group of bivalent groups listed below 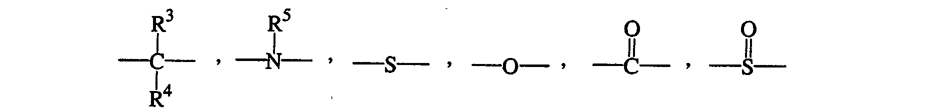 (where, in the formula, R³, R⁴, and R⁵ each independently denote a hydrogen atom, alkyl group with 1-24 carbon atoms, aryl group, arylalkyl group, or hydroxy group).

As one embodiment of the present invention, the cosmetic-use polymer wherein said guanidino group-comprising repeating unit is denoted by general formula (II) below, is provided. 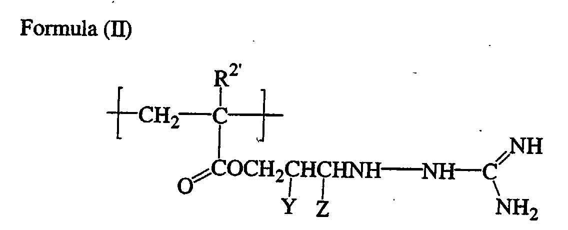 (where, in the formula, R^{2'} denotes a hydrogen atom or methyl group, one from among Y and Z denotes a hydrogen atom and the other denotes a hydroxy group, and the guanidino group in the formula is optionally a salt to which an acid has been added).

As one prefer embodiment, the cosmetic-use polymer comprising not less than 5 weight percent (more prefer, 15 weight percent) of said guanidino group-comprising (including a salt with an added acid) repeating unit.

As one embodiment, the cosmetic-use polymer further comprising a repeating unit derived from the compound denoted by general formula (V) below 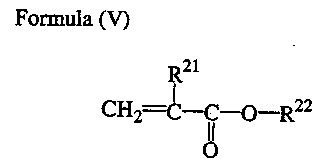 (where, in formula (V), R²¹ denotes a hydrogen atom or methyl group and R²² denotes an alkyl group with 1-24 carbon atoms).

The foregoing objects are accomplished by the invention to provide a cosmetic comprising a polymer having a weight average molecular weight of 5.0·10³-1.0·10⁷ and comprising a repeating unit having at least the guanidino group denoted by general formula (I) below 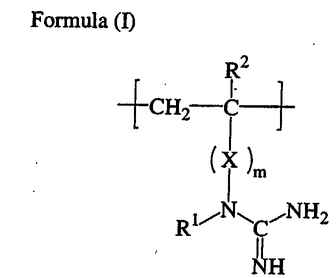 (where, in the formula, R¹ denotes a hydrogen atom or hydrocarbon group, R² denotes a hydrogen atom or methyl group, X denotes a bivalent connecting group, m denotes 0 or 1, and the guanidino group in the formula is optionally a salt to which an acid has been added).

As one embodiment, said cosmetic in the form of a hair cosmetic, a skin cosmetic or a nail cosmetic is provided. As one prefer embodiment, said cosmetic in the form of a hair fixative is provided.

As one embodiment, the skin or nail cosmetic comprising at least one member selected from the group consisting of water, alcohol solvents, ester solvents, ketone solvents, and hydrocarbon solvents is provided.

As one embodiment, the hair cosmetic comprising at least one member selected from the group consisting of water and alcohol solvents is provided.

From another perspective, the present invention provides a method of treating keratinous substances comprising the step of applying to a keratinous substance a guanidino group-comprising polymer having a weight average molecular weight of 5.0·10³-1.0·10⁷ and having a repeating unit comprising at least the guanidino group denoted by said general formula (I); a method of using as a cosmetic a guanidino group-comprising polymer having a weight average molecular weight of 5.0·10³-1.0·10⁷ and having a repeating unit comprising at least the guanidino group denoted by said general formula (I); and a method of preparing a cosmetic comprising the step of dissolving in a solvent, emulsifying, and/or dispersing a guanidino group-comprising polymer having a weight average molecular weight of 5.0·10³-1.0·10⁷ and having a repeating unit comprising at least the guanidino group denoted by said general formula (I).

From another perspective, the present invention provides a method of preparing a polymer comprising a repeating unit having at least the guanidino group denoted by said general formula (I), comprising the step of preparing a monomer having at least a guanidino group (including a salt with an added acid) and the step of polymerizing said monomer alone or with another monomers; the method of preparing said polymer comprising the step of polymerizing a nitrogen-comprising monomer alone or with another monomers to obtain a nitrogen-comprising polymer and the step of guanidinizing said nitrogen-comprising polymer; and the method of preparing said polymer comprising the step of polymerizing a monomer having a reactive functional group alone or with another monomers to obtain a polymer having a reactive functional group and the step of reacting said polymer with a compound having both a guanidino group and a reactive group capable of reacting with said functional group.

### Detailed Description of the Invention

The present invention is described in detail below. In the present *Specification*, the symbol "-" indicates a range having as minimum and maximum the two numbers before and after it, inclusive.

### (Cosmetic-Use Polymer)

The cosmetic-use polymer of the present invention (also referred to hereinafter as a "guanidino group-comprising polymer") is characterized by comprising at least the repeating unit denoted by general formula (I) below and by having a weight average molecular weight of 5.0·10³-1.0·10⁷.

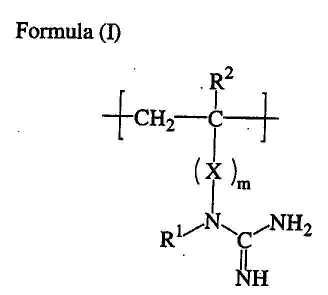

In the formula, R¹ denotes a hydrogen atom or hydrocarbon group, R² denotes a hydrogen atom or methyl group, X denotes a bivalent connecting group, and m denotes 0 or 1. However, the guanidino group in the formula may also be a salt to which an acid has been added.

The hydrocarbon group denoted by R¹ comprises a substituted or unsubstituted alkyl group and a substituted or unsubstituted aryl group. R¹ is desirably a hydrogen atom or an alkyl group with 1-4 carbon atoms.

In general formula (I) above, X denotes a bivalent connecting group and m denotes 1 or 0. Preferably, m is 1. X is desirably one or a combination of two or more connecting groups selected from among the group consisting of the bivalent groups listed below. When a polymer having no those connecting groups is employed, for example, in a hair cosmetic, setting strength tends to be poor, flexibility decreases, and a feeling of tackiness results.

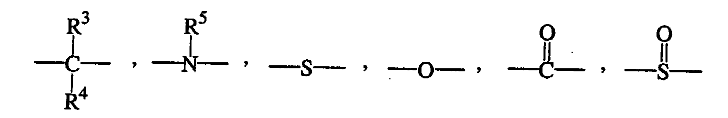

In the formula, R³ and R⁴ each denote a hydrogen atom, an alkyl group with 1-24 carbon atoms, an aryl group, an arylalkyl group, or a hydroxy group. R⁵ denotes a hydrogen atom, an alkyl group with 1-24 carbon atoms, an aryl group, or an arylalkyl group.

The above-described guanidino group-comprising polymer has a weight average molecular weight of 5.0·10³-1.0·10⁷. When the weight average molecular weight of the guanidino group-comprising polymer is less than 5.0·10³, the film forming property and film durability decrease. When the weight average molecular weight exceeds 1.0·10⁷, solubility in the water employed as solvent decreases and solution viscosity increases, resulting in problems when used as a cosmetic. The preferred range of the weight average molecular weight of the above-described guanidino-comprising polymer varies with the application, but when employed as a hair cosmetic, the weight average molecular weight is desirably 2.0·10⁴-1.5·10⁵. When employed as a skin cosmetic, the weight average molecular weight is desirably 1.0·10⁴-5·10⁵, and when employed as a nail cosmetic, the weight average molecular weight is desirably 2.0·10⁴-1.0·10⁶.

The guanidino group contained in the guanidino group-comprising polymer may be a salt to which an acid has been added. The acid capable of forming an acid adduct salt with the guanidino group may be an organic or inorganic acid, examples of which are: formic acid, acetic acid, propionic acid, butyric acid, isobutyric acid, hexanoic acid, heptanoic acid, octanoic acid, nonanoic acid, decanoic acid, lauric acid, myristic acid, palmitic acid, stearic acid, acrylic acid, methacrylic acid, crotonic acid, isocrotonic acid, phenyl acetate, cinnamic acid, benzoic acid, sorbic acid, nicotinic acid, urocanic acid, pyrrolidone carboxylic acid, and other monocarboxylic acids; oxalic acid, malonic acid, succinic acid, glutamic acid, adipic acid, pimelic acid, cork acid, azelaic acid, sebacic acid, maleic acid, fumaric acid, phthalic acid, terephthalic acid, and other dicarboxylic acids; glucolic acid, lactic acid, malic acid, tartaric acid, citric acid, o, m, and p-hydroxybenzoic acid, and other hydroxy acids; glycine, alanine, β-alanine, valine, leucine, phenylalanine, tyrosine, serine, threonine, methionine, cysteine, cystine, proline, hydroxyproline, pipecolic acid, tryptophan, aspartic acid, asparagine, glutamic acid, glutamine, lysine, histidine, ornithin, arginine, aminobenzoic acid, and other amino acids; methanesulfonic acid, trifluoromethanesulfonic acid, and other lower alkyl sulfonic acids; benzenesulfonic acid, p-toluenesulfonic acid, and other aryl sulfonic acids; hydrofluoric acid, hydrochloric acid, hydrobromic acid, hydroiodic acid, and other halogenated hydroacid; and perchloric acid, sulfuric acid, nitric acid, phosphoric acid, carbonic acid, and other inorganic acids.

The preferred embodiment of implementing the cosmetic-use polymer of the present invention has a repeating unit comprising at least the guanidino group denoted by general formula (II) below.

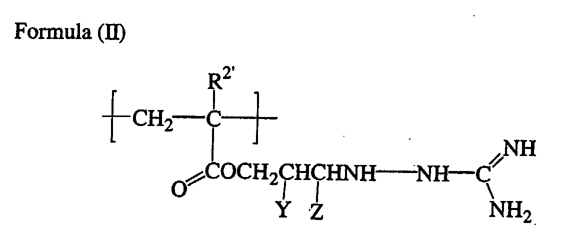

In general formula (II), R^{2'} denotes a hydrogen atom or methyl group and one from among Y and Z denotes a hydrogen atom while the other denotes a hydroxy group. The guanidino group in the formula may be a salt to which an acid has been added.

In the present invention, the guanidino group-comprising polymer may comprise a hydrophobic unit and/or a hydrophilic unit in addition to the repeating unit having a guanidino group.

Examples of such hydrophobic units are esters of alcohols having 1-24 carbon atoms with (meth)acrylic acid, styrene, p-methylstyrene, p-chlorostyrene, vinylmethylether, vinylcyclohexylether, vinyl acetate, diethyl maleate, dibutyl maleate, and other hydrophobic vinyl monomers, glycidyl (meth)acrylate (the term "(meth)acrylate" will hereinafter embrace both acrylate and methacrylate), fluoroalkylesters of (meth)acrylic acid, N-octylacrylamide, and other mono- or disubstituted (meth)acrylamide derivatives having alkyl groups with 4-24 carbon atoms, and other repeating units derived from vinyl-comprising macromonomers.

Examples of the above-mentioned esters of alcohols having 1-24 carbon atoms with (meth)acrylic acid are methyl (meth)acrylate, ethyl (meth)acrylate, propyl (meth)acrylate, isopropyl (meth)acrylate, butyl (meth)acrylate, isobutyl (meth)acrylate, sec-butyl (meth)acrylate, tert-butyl (meth)acrylate, pentyl (meth)acrylate, sec-pentyl (meth)acrylate, 1-ethylpropyl (meth)acrylate, 2-methylbutyl (meth)acrylate, isopentyl (meth)acrylate, tert-pentyl (meth)acrylate, 3-methylbutyl (meth)acrylate, neopentyl (meth)acrylate, hexyl (meth)acrylate, 2-methylpethyl (meth)acrylate, 4-methylpentyl (meth)acrylate, 2-ethylbutyl (meth)acrylate, cyclopentyl (meth)acrylate, cyclohexyl (meth)acrylate, heptyl (meth)acrylate, 2-heptyl (meth)acrylate, 3-heptyl (meth)acrylate, octyl (meth)acrylate, 2-octyl (meth)acrylate, 2-ethylhexyl (meth)acrylate, isooctyl (meth)acrylate, nonyl (meth)acrylate, 3,3,5-trimethylhexyl (meth)acrylate, decyl (meth)acrylate, undecyl (meth)acrylate, lauryl (meth)acrylate, cetyl (meth)acrylate, stearyl (meth)acrylate, eicosyl (meth)acrylate, docosyl (meth)acrylate, tetracosyl (meth)acrylate, methylcyclohexyl (meth)acrylate, isobornyl (meth)acrylate, norbornyl (meth)acrylate, benzyl (meth)acrylate, and phenethyl (meth)acrylate.

Of these, repeating units derived from the compound denoted by general formula (V) are preferred as hydrophobic units.

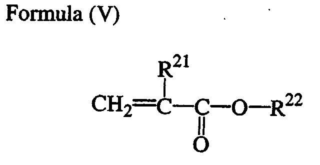

In general formula (V), R²¹ denotes a hydrogen atom or a methyl group and R²² denotes an alkyl group with 1-24 carbon atoms. That is, the compound denoted by general formula (V) is an ester of an alcohol having 1-24 carbon atoms and (meth)acrylic acid.

Examples of the hydrophilic unit combined with the repeating unit comprising the guanidino group are repeating unit derived from hydrophilic monomers that are nonionic, anionic, cationic, or amphoteric, that is, exhibit both anionic and cationic properties within a single molecule.

Specific examples of nonionic monomers among the above-listed hydrophilic monomers are: (meth)acrylonitrile, N-cyclohexylmaleimide, N-phenylmaleimide, N-vinylpyrrolidone, N-(meth)acryloylmorpholine, hydroxyethyl (meth)acrylate, polyethyleneglycol (meth)acrylate, methoxyethyl (meth)acrylate, ethoxyethyl (meth)acrylate, methoxypoly(ethyleneglycol/propyleneglycol) mono(meth)acrylate, polyethyleneglycol di(meth)acrylate, N-polyalkyleneoxy(meth)acrylamide, other monomers derived from alkyleneoxides with 2-4 carbon atoms of (meth)acrylic acid or (meth)acrylamide, N-methylacrylamide, N-isopropylmethacrylamide, N,N-dimethylmethacrylamide, other acrylamides, and N-vinyl-e-caprolactam.

Specific examples of anionic monomers are: (meth)acrylic acid, maleic acid, anhydrous maleic acid, itaconic acid, fumaric acid, crotonic acid, and other unsaturated carboxylic acid monomers; half esters of unsaturated polybasic acid anhydrides (such as succinic anhydride and phthalic anhydride) and hydroxyl group-comprising (meth)acrylates such as hydroxyethyl (meth)acrylate; monomers having sulfonic acid groups such as styrene sulfonate and sulfoethyl (meth)acrylate; and monomers having phosphoric acid groups such as acid phosphoxyethyl (meth)acrylate. These anionic monomers may be employed without alteration as acids, or partially or completely neutralized.

Examples of bases suitable for use in neutralization are potassium hydroxide, sodium hydroxide, other alkali metal hydroxides, ammonia water, (mono-, di-, or tri-) ethanolamines, trimethylamines, and other amine compounds.

Specific examples of cationic monomers are: cationized N,N-dimethylaminoethyl (meth)acrylate, N,N-diethylaminoethyl (meth)acrylate, N,N-dimethylaminopropyl (meth)acrylate, N,N-diethylaminopropyl (meth)acrylate, N,N-dimethylaminoethyl (meth)acrylamide, N,N-diethylaminoethyl (meth)acrylamide, N,N-dimethylaminopropyl (meth)acrylamide, N,N-diethylaminopropyl (meth)acrylamide, p-dimethylaminomethylstyrene, p-dimethylaminoethylstyrene, p-diethylaminomethylstyrene, and p-diethylaminoethylstyrene monomers. Examples of cationizing agents are methyl chloride, methyl bromide, methyl iodide, and other alkyl halides; dimethyl sulfate and other dialkyl sulfates; epichlorhydrin adducts of N-(3-chloro-2-hydroxypropyl)-N,N,N-trimethylammonium chloride and other tertiary amine inorganic acid salts; hydrochloric acid, hydrobromic acid, sulfuric acid, phosphoric acid, and other inorganic acids; and formic acid, acetic acid, propionic acid, and other carboxylic acids.

Monomers obtained by treating the above-listed cationic monomers with a modifying agent such as a sodium or potassium haloacetate may be employed as amphoteric monomers.

In the present invention, the guanidino group-comprising polymer incorporates at least a repeating unit having a guanidino group produced or introduced before or after polymerization. Of the total number of repeating units comprised by the guanidino comprising-polymer, the proportion of repeating units having the guanidino group is desirably at least 5 weight percent, preferably not less than 15 weight percent. Further, in the guanidino group-comprising polymer, there is no specific upper limit to the quantity of repeating units having a guanidino group; a desirable range can be established for each use. For example, when a hair cosmetic-use polymer is employed in a hair fixative, the repeating units comprising guanidino groups desirably comprise 80 weight percent or less. When employed in a conditioning agent (for example, a shampoo and a conditioner), the proportion of guanidino-group comprising repeating units is desirably not greater than 90 weight percent.

The guanidino group-comprising polymer may also comprise hydrophobic units, but in uses where water solubility is required (such as in hair cosmetics), the hydrophobic unit is desirably incorporated in a range that does not reduce water solubility. From this perspective, of the total number of repeating units comprising the guanidino group-comprising polymer, hydrophobic units desirably comprise not more than 85 weight percent, preferably 10-85 weight percent. When the proportion of hydrophobic units exceeds 85 weight percent, the smoothness and transparency of the film of guanidino group-comprising polymer decreases, its water solubility decreases, and its removal during hair washing sometimes becomes difficult.

When the guanidino group-comprising polymer contains the above-mentioned hydrophilic units, the hydrophilic units desirably comprise not more than 50 weight percent of the guanidino-comprising repeating units making up the guanidino group-comprising polymer.

### (Method of Preparing Guanidino group-comprising polymer)

Next, the method of preparing the cosmetic-use polymer of the present invention, that is, guanidino group-comprising polymer, will be described.

The guanidino group-comprising polymer of the present invention may be manufactured by polymerizing a monomer comprising a guanidino group (referred to as a "guanidino group-comprising monomer" hereinafter) either alone or with another monomer. Preferred examples of guanidino group-comprising monomers are the monomers denoted by general formula (III) below, and particularly preferred examples are the monomers denoted by general formula (III-a) below.

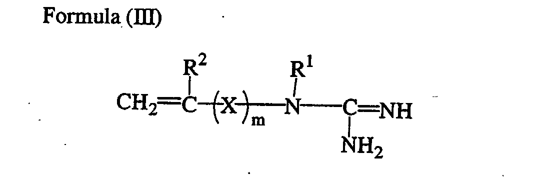

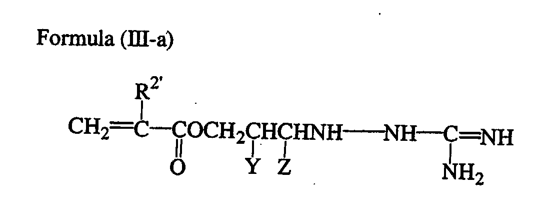

In general formula (III), R¹, R², X, and m are defined as in general formula (I) above and the preferred ranges thereof are identical. In general formula (III-a), R^{2'}, Y, and Z are defined as in general formula (II) above.

The guanidino group-comprising monomer may be in the form of an acid adduct where an acid is added to the guanidino group. The acid capable of forming an acid adduct salt with the guanidino group-comprising monomer may be an organic or inorganic acid, examples of which are: formic acid, acetic acid, propionic acid, butyric acid, isobutyric acid, hexanoic acid, heptanoic acid, octanoic acid, nonanoic acid, decanoic acid, lauric acid, myristic acid, palmitic acid, stearic acid, acrylic acid, methacrylic acid, crotonic acid, isocrotonic acid, phenyl acetate, cinnamic acid, benzoic acid, sorbic acid, nicotinic acid, urocanic acid, pyrrolidone carboxylic acid, and other monocarboxylic acids; oxalic acid, malonic acid, succinic acid, glutamic acid, adipic acid, pimelic acid, cork acid, azelaic acid, sebacic acid, maleic acid, fumaric acid, phthalic acid, terephthalic acid, and other dicarboxylic acids; glucolic acid, lactic acid, malic acid, tartaric acid, citric acid, o, m, and p-hydroxybenzoic acid, and other hydroxy acids; glycine, alanine, β-alanine, valine, leucin, phenylalanine, tyrosine, serine, threonine, methionine, cysteine, cystine, proline, hydroxyproline, pipecolic acid, tryptophan, aspartic acid, asparagine, glutamic acid, glutamine, lysine, histidine, ornithin, arginine, aminobenzoic acid, and other amino acids; methanesulfonic acid, trifluoromethanesulfonic acid, and other lower alkyl sulfonic acids; benzenesulfonic acid, p-toluenesulfonic acid, and other aryl sulfonic acids; hydrofluoric acid, hydrochloric acid, hydrobromic acid, hydroiodic acid, and other halogenated hydroacid; and perchloric acid, sulfuric acid, nitric acid, phosphoric acid, carbonic acid, and other inorganic acids.

The guanidino group-comprising monomer can be manufactured by reacting a monomer having a reactive functional group with a compound having both of a guanidino group and a reactive group capable of reacting with the functional group (also referred to below as a "guanidino group-introducing reagent"). Specifically, the guanidino group-comprising monomer can be manufactured by reacting a monomer having an epoxy group such as glycidyl (meth)acrylate with a guanidino group-introducing reagent having both a reactive group capable of reacting with the epoxy group such as a reactive amino group or carboxyl group and a guanidino group; by reacting an acid anhydride such as maleic anhydride with a guanidino group-introducing reagent having both a reactive group such as a hydroxy group or amino group capable of reacting with the acid anhydride and a guanidino group; by reacting methacrylic acid with a guanidino group-introducing reagent having both a reactive group such as a hydroxyl group, methoxy group, or epoxy group capable of reacting with methacrylic acid and a guanidino group; and by reacting methacrylic acid with a guanidino group-introducing reagent having both a guanidino group and a functional group capable of exchanging esters with methacrylic acid.

Examples of guanidino group-introducing reagents comprising both a reactive group and a guanidino group are: aminoguanidine(NH₂-NH-C(=NH)(NH₂)), hydroxyguanidine, (2-hydroxypropyl)guanidine, (4-hydroxybutyl)guanidine, (5-hydroxypentyl)guanidine, (6-hydroxyhexyl)guanidine, 2-(2-hydroxyehtoxy)ethylguanidine, 2-[2-(2-hydroxyethoxy)ethoxy]ethylguanidine, 1-(3-hydroxypropyl)-1-methylguanidine, 1-(2-hydroxypropyl)-1-methylguanidine, 1-(4-hydroxybutyl)-1-methylguanidine, 1-(5-hydroxypentyl)-1-methylguanidine, 1-(6-hydroxyhexyl)-1-methylguanidine, 1-[2-(2-hydroxyethoxy)ethyl]-1-methylguanidine, 1-[2-(2-(2-hydroxyethoxy)ethoxy)ethyl]-1-methylguanidine, 1,1-bis(2-hydroxyethyl)guanidine, 1,1-bis(3-hydroxypropyl)guanidine, 1,1-bis(2-hydroxypropyl)guanidine, 1,1-bis(4-hydroxybutyl)guanidine, 1,1-bis(5-hydroxypentyl)guanidine, 1,1-bis(6-hydroxyhexyl)guanidine, 1,1-bis[2-(2-hydroxyethoxy)ethyl]guanidine, 1,1-bis[2-(2-(2-hydroxyehtoxy)ethoxy)ethyl]guanidine, 2-guanidinoethyl acetate, 3-guanidinopropyl acetate, 2-guanidino-2-propyl acetate, 4-guanidine-1-butyl acetate, 5-guanidine-1-pentyl acetate, 6-guanidino-1-hexyl acetate, 2-(2-guanidinoethoxy)ethyl acetate, 2-[2-(2-guanidinoethoxy)ethoxy]ethyl acetate, 2-(1-methylguanidino)ethyl acetate, 3-(1-methylguanidino)propyl acetate, 2-(1-methylguanidino)-1-methylethyl acetate, 4-(1-methylguanidino)butyl acetate, 5-(1-methylguanidino)pentyl acetate, 6-(1-methylguanidino)pentyl acetate, 2-[2-(1-methylguanidino)ethoxy]ethyl acetate, 2-[2-(2-(1-methylguanidino)ethoxy)ethoxy]ethyl acetate, 2-guanidinoethyl benzoate, 3-guanidinopropyl benzoate, 2-guanidino-2-propyl benzoate, 4-guanidino-1-butyl benzoate, 5-guanidino-1-pentyl benzoate, 6-guanidino-1-hexyl benzoate, 2-(2-guanidinoethoxy)ethyl benzoate, 2-[2-(2-guanidinoethoxy)ethoxy]ethyl benzoate, 2-(1-methylguanidino)ethyl benzoate, 3-(1-methylguanidino)propyl benzoate, 2-(1-methylguanidino)-1-methylethyl benzoate, 4-(1-methylguanidino)butyl benzoate, 5-(1-methylguanidino)pentyl benzoate, 6-(1-methylguanidino)pentyl benzoate, 2-[2-(1-methylguanidino)ethoxy]ethyl benzoate, 2-[2-(2-(1-methylguanidino)ethoxy)ethoxy]ethyl benzoate, 2-guanidinoethyl salicylate, 3-guanidinopropyl salicylate, 2-guanidino-2-propyl salicylate, 4-guanidino-1-butyl salicylate, 5-guanidino-1-pentyl salicylate, 6-guanidino-1-hexyl salicylate, 2-(2-guanidinoethoxy)ethyl salicylate, 2-[2-(2-guanidinoethoxy)ethoxy]ethyl salicylate, 2-(1-methylguanidino)ethyl salicylate, 3-(1-methylguanidino)propyl salicylate, 2-(1-methylguanidino)-1-methylethyl salicylate, 4-(1-methylguanidino)butyl salicylate, 5-(1-methylguanidino)pentyl salicylate, 6-(1-methylguanidino)pentyl salicylate, 2-[2-(1-methylguanidino)ethoxy]ethyl salicylate, 2-[2-(2-(1-methylguanidino)ethoxy)ethoxy]ethyl salicylate, 2-guanidinoethyl m- and p-hydroxybenzoate, 3-guanidinopropyl m- and p-hydroxybenzoate, 2-guanidino-2-propyl m- and p-hydroxybenzoate, 4-guanidino-1-butyl m- and p-hydroxybenzoate, 5-guanidino-1-pentyl m- and p-hydroxybenzoate, 6-guanidino-1-hexyl m- and p-hydroxybenzoate, 2-(2-guanidinoethoxy)ethyl m- and p-hydroxybenzoate, 2-[2-(2-guanidinoethoxy)ethoxy]ethyl m and p-hydroxybenzoate, 2-(1-methylguanidino) ethyl m- and p-hydroxybenzoate, 3-(1-methylguanidino)propyl m- and p-hydroxybenzoate, 2-(1-methylguanidino)-1-methylethyl m- and p-hydroxybenzoate, 4-(1-methylguanidino)butyl m- and p-hydroxybenzoate, 5-(1-methylguanidino)pentyl m- and p-hydroxybenzoate, 6-(1-methylguanidino)pentyl m- and p-hydroxybenzoate, 2-[2-(1-methylguanidino)ethoxy]ethyl m- and p-hydroxybenzoate, 2-[2-(2-(1-methylguanidino)ethoxy)ethoxy]ethyl m- and p-hydroxybenzoate, L-arginine and derivatives thereof, guanidinopropanoate, guanidinoethyl phosphate, and the like.

The guanidine compound denoted by general formula (III), or an acid adduct salt thereof, can be obtained by the preparing methods indicated by the following reaction equation. That is, the compound denoted by general formula (III), or an acid adduct salt thereof, can be obtained by reacting a guanidinizing reagent with the nitrogen-comprising monomer denoted by general formula (IV). In the reaction equations below, X, R¹, R², and m are defined as in general formula (I) above.

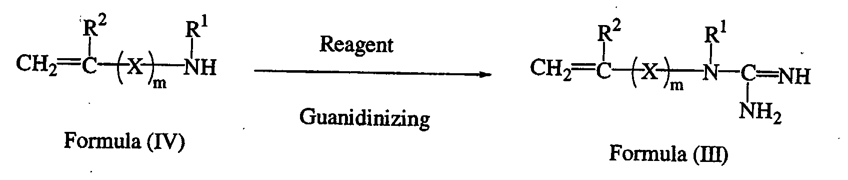

In the above reaction equation, examples of the nitrogen-comprising monomer starting material denoted by general formula (IV) are: the reaction product of an unsaturated group-comprising acid anhydride such as maleic anhydride, itaconic anhydride, or crotonic anhydride with a compound having a reactive group capable of reacting with these anhydrides, such as diamine derivatives and aniline; the reaction product of an epoxy group-comprising compound such as glycidyl methacrylate and a compound having a reactive group capable of reacting with the epoxy group, such as a diamine derivative; the reaction product of an epoxy group-comprising compound such as glycidyl methacrylate and a compound having a reactive group capable of reacting with the epoxy group, such as hydroxyl group-comprising amine derivatives; and the reaction product of isocyanate group-comprising compounds such as 2-isocyanate ethyl (meth)acrylate and a compound having a reactive group capable of reacting with the isocyanate group, such as a hydroxyl group-comprising amine derivative.

The various reagents conventionally employed in guanidinization reactions may be broadly employed as the guanidinizing reagent. Examples are: cyanamide, S-alkylisothiourea, O-alkylisourea, aminoiminomethanesulfonic acid, 3,5-dimethyl-1-guanylpyrazole, and 1H-pyrazole-1-carboamidine.

When employing S-alkylisothiourea, O-alkylisourea, 3,5-dimethyl-1-guanylpyrazole, or 1H-pyrazole-1-carboamidine as the guanidinizing agent, the reaction is desirably conducted in the presence of a base. Bases suitable for use in the reaction system are barium hydroxide, calcium hydroxide, magnesium hydroxide, sodium hydroxide, potassium hydroxide, lithium hydroxide, sodium bicarbonate, sodium carbonate, ammonia water, tertiary amines (for example, triethylamine, N,N-dimethylaniline, N,N-dimethylpiperazine, and N-methylpiperazine), and pyridine. The reaction is desirably conducted for 1-72 hrs at 25-200°C with stirring. When cyanamide is employed as the guanidinizing reagent, the reaction is desirably conducted for 1-72 hrs at 0-100°C with stirring. The reaction can also be conducted for 1-72 hrs at 25-200°C in the presence of the acids given as examples for acid adducts of guanidine instead of a base. Further, once the reaction has ended, an acid can be added by the usual methods as needed to isolate the compound as an acid adduct salt.

A radical polymerization initiator is normally employed as the polymerization initiator during preparing of the guanidino group-comprising polymer of the present invention. The polymerization method is not specifically limited. Solution polymerization, bulk polymerization, suspension polymerization, and various other methods of polymerization may be employed. However, the use of solution polymerization is preferred. Solvents suitable for use in polymerization are: acetone, methyl ethyl ketone, methyl isobutyl ketone, methanol, ethanol, propanol, isopropanol, butanol, isobutanol, sec-butanol, ethyl acetate, propyl acetate, butyl acetate, and other organic solvent. These organic solvents may be employed singly or in combinations of two or more.

Examples of radical polymerization initiators suitable for use during polymerization are: 2,2'-azobisisobutyronitrile (AIBN), 2,2'-azobis(2,4-dimethylvaleronitrile), 2,2'-azobis(4-methoxy-2,4-dimethylvaleronitrile), dimethyl-2,2'-azobisisobutyrate, 2,2'-azobis(2-methylbutyronitrile), 1,1'-azobis(1-cyclohexanecarbonnitrile), and other azo compounds, benzoyl peroxide, dicumyl peroxide, di-t-butyl peroxide, lauroyl peroxide, and other peroxide compounds. These polymerization initiators are usually employed in a proportion of 0.01-5 weight percent based on the total amount of all monomer components.

Polymerization is normally conducted for 1-20 hrs at 30-120°C, preferably 40-100°C, in an inert gas atmosphere of nitrogen or argon.

The above-described hydrophobic and hydrophilic monomers may be employed in combination with the guanidino group-comprising monomer.

Further, the guanidino group-comprising polymer of the present invention may be manufactured by polymerizing of nitrogen-comprising monomers either alone or with another monomers, followed by guanidinizing the nitrogen contained in the nitrogen-comprising monomer. Guanidinization is conducted by adding a guanidinizing reagent to a polymer solution obtained by polymerizing the nitrogen-comprising monomer and stirring and/or heating as desired. The same guanidinizing reagents and the nitrogen-comprising monomers given above as examples suitable for use in preparing guanidino group-comprising monomer may be employed as the above-mentioned guanidinizing reagent and nitrogen-comprising monomer. The guanidinizing reaction may also be conducted based on the above-described examples of preparing guanidino group-comprising monomers.

The above-described hydrophobic and hydrophilic monomers are examples of other monomers suitable for copolymerization with the above-described nitrogen-comprising monomers.

Further, the guanidino group-comprising polymer of the present invention can be manufactured by polymerizing a monomer having a reactive functional group either alone or with another monomer, and then reacting the product with a compound (guanidino group-introducing compound) having both a reactive group capable of reacting with the functional group and a guanidino group. For example, preparing is possible by polymerizing a monomer having an epoxy group such as glycidyl (meth)acrylate and then reacting the product with a guanidino group-introducing reagent having both a reactive group such as an amino group or carboxyl group capable of reacting with the epoxy group and a guanidino group; polymerizing an acid anhydride such as maleic anhydride and then reacting the product with a guanidino group-introducing agent having both a reactive group such as a hydroxyl group or amino group capable of reacting with the acid anhydride and a guanidino group; polymerizing methacrylic acid and then reacting the product with a guanidino group-introducing reagent having both a reactive group such as a hydroxyl group, methoxy group, or epoxy group capable of reacting with the acid and a guanidino group; and polymerizing methacrylic acid and then reacting the product with a guanidino group-introducing reagent having a functional group capable of exchanging esters with methacrylic acid and a guanidino group.

The above-described hydrophobic and hydrophilic monomers are examples of other monomers capable of copolymerization with the above-mentioned monomer having a reactive functional group.

The guanidino group can be introduced by adding a guanidino group-introducing reagent to a polymer solution obtained by polymerizing monomer components, and reacting the mixture for 0.1-100 hr, preferably 1-50 hr, at 20-100°C. The guanidino group-introducing reagent is desirably employed in a proportion of 0.2-3 times, preferably 0.5-2 times, the molar equivalent of the reactive functional group (for example, epoxy group) contained in the polymer of precursor monomer (for example, glycidyl methacrylate). The guanidino group-introducing reagents given as examples suitable for use in the preparing of the guanidino group-comprising monomer can be employed as the guanidino group-introducing reagent.

The guanidino group-comprising polymer manufactured by the above-described methods can be employed for various applications either as is in the polymer solution without separation, or following separation by a known method such as reprecipitation or solution distillation. The separated guanidino group-comprising polymer can be refined as needed by reprecipitation, solvent washing, membrane isolation, and adsorption processing, and then employed to prepare cosmetics.

### (Cosmetics)

Hair, skin, and nail cosmetics employing the cosmetic-use polymer of the present invention will be described next. The present invention also provides methods of employing the above-described cosmetic-use polymer in cosmetics. The cosmetics of the present invention can be manufactured, for example by dissolving, emulsifying, and/or dispersing the cosmetic-use polymer of the present invention and other additives in a solvent.

The hair, skin, and nail cosmetics of the present invention may comprise, in addition to the above-described essential component in the form of the cosmetic-use polymer of the present invention, natural or synthesized cationic, anionic, nonionic, or amphoteric polymers, as well as modified products thereof.

Examples of the cationic polymer include, as examples of the synthetic cationic polymer, N-vinyl pyrrolidone/quatemized dimethylaminoethyl methacrylate copolymers such as "Gafquat 755N, 755 and 734N" (each, trade name; product of ISP, Inc.) and "Ruviquat PQ11" (trade name; product of BASF AG); N-vinyl pyrrolidone/dimethylaminoethyl methacrylate copolymers such as "Copolymer 845, 937 and 958" (each, trade name; product of ISP, Inc.); N-vinyl pyrrolidone/N-vinyl caprolactam/dimethylaminoethyl methacrylate copolymers such as "Gafix VC-713" (trade name; product of ISP, Inc.);N-vinyl pyrrolidone/methacrylamidopropyl trimethylammonium chloride copolymer such as "Gafquat HS-100" (trade name; product of ISP, Inc.); N-vinyl pyrrolidone/quatemized methylvinylimidazolium copolymer such as "Luviquat FC370, FC550, FC905 and HM-552" (each, trade name; product of BASF AG); dimethyldiallylammonium chloride polymers and dimethyldiallylammonium chloride/acrylamide copolymers such as "Merquat 100 and 550" (trade name; Calgon); and quaternized dialkylaminoalkylene methacrylate/alkyl(meth)acrylate copolymers as disclosed in JP-A-4-21623 or JP-A-5-310538.

Examples of the cationic polymer obtained by modifying a natural product include hydroxyethyl cellulose/dimethyldiallyl ammonium chloride copolymers such as "Cellquat H-100 and L-200" (each, trade name; product of National Starch & Chemical Co.); reaction products of hydroxyethyl cellulose with an epoxylated trimethyl ammonium compound such as "Cellquat SC-240, SC-240C and SC-230M" (each, trade name; product of National Starch & Chemical Co.), "Ucare Polymer JR-125, JR-400 and JR-30M" (each, trade name; product of Amerchol); "Reoguard G" (trade name; product of Lion Corp.) and "Catinal HC and LC" (trade name; product of Toho Chemical Industry Co.); and quaternized chitosan such as "kytamer PC" (trade name; product of Amerchol).

In the cosmetic composition of the present invention, the weight ratio of the guanidino group-comprising polymer to the cationic polymer preferably falls within a range from 1:10 to 10:1, more preferably from 1:5 to 10:1. When the ratio is less than 1:10, in the case of hair cosmetics, the hair setting effects tends to be insufficient. This prevents the retention of a desired hair style, and hair that is set with such a composition does not have sufficient resilience under high humidity conditions and become tacky. Moreover, repeated use of the composition over a long time period causes problems such as built-up. When the ratio exceeds 10:1, in the case of hair cosmetics, hair that is set with the composition tends to be difficult to be combed due to lack of a slick feeling, and moreover, the smoothness after drying tends to be insufficient. In the case of hair cosmetics, the total amount of the guanidino group-comprising polymer and the cationic polymers is preferably 0.1-10 wt. %, more preferably 0.5-8 wt. %, based on the total amount of hair cosmetics. Amounts of less than 0.1 wt. % lead to insufficient hair setting property, while those exceeding 10 wt. % tend to increase stiffness and deteriorate the touch feeling.

Examples of anionic polymers include polymers having an acid group such as carboxyl or sulfonic acid group. Specific examples include methyl vinyl ether/maleic anhydride alkyl half ester copolymers such as "Gantrez ES-225, ES-425, A-425, V-225 and V-425" (each, trade name; product of IPS, Inc.); vinyl acetate/crotonic acid copolymers such as "Resin 28-131" (trade name; product of National Starch & Chemical Co.) and "Luviset CA" (trade name; product of BASF AG); vinyl acetate/crotonic acid/vinyl neodecanoate copolymers such as "Resin 28-2930" (trade name; product of National Starch & Chemical Co.); vinyl acetate/monobutyl maleate/isobornyl acrylate copolymers such as "ADVANTAGE CP" (trade name; product of ISP, Inc.); (meth)acrylic acid/(meth)acrylic ester copolymers such as "Ruvimer 100P" (trade name; product of BASF AG); acrylic acid/acrylamide derivative copolymers such as "Ultrahold Strong and Ultrahold 8" (each, trade name; product of BASF AG), "Versatile 42" (trade name; product of National Starch & Chemical Co.) and "Plus Size L53P" (trade name; product of GOO Chemical); polyvinyl pyrrolidone/(meth)acrylic acid/(meth)acrylic ester copolymers such as "Luviflex VBM35" (tarde name; Product of BASF AG); and diethylene glycol/cyclohexane dimethanol/dimethyl isophthalate/sulfonated dimethyl isophthalate condensates such "Eastman AQ Polymer" (trade name; product of Eastman Chemical).

From the viewpoint of water solubility, the anionic copolymers is preferabaly used after its acid group is partially or wholly neutralized with a basic compound. Examples of such a basic compound include hydroxide of an alkali metal such as sodium hydroxide and potassium hydroxide; inorganic basic compounds such as aqueous ammonia; alkanolamines such as ethanolamine, diethanolamine, triethanolamine, triisopropanolamine, 2-amino-2-methyl-1-propanol, 2-amino-2-methyl-1,3-propandiol and aminomercaptopropanediol; and basic amino acid compounds such as lysine, arginine and histidine. Among them, 2-amino-2-methyl-1-propanol and potassium hydroxide are preferred from the viewpoint of water solubility.

The weight ratio of the guanidino group-comprising polymer to the anionic polymer preferably falls within a range from 1:10 to 10:1, more preferably from 1:5 to 10:1. When the ratio is less than 1:10, in the case of the hair cosmetics, the resulting cosmetic composition tends to have insufficient flexibility, make the hair stiff and provide insufficient hair setting effects. This leads to flaking upon combing, and difficulty in setting the hair to a desired style due to static electricity. When the ratio exceeds 10:1, the resulting composition does not impart sufficient hardness so that the hair thus set lacks tautness and has a heavy (deterionated) finish feeling. The total amount of the guanidino group-comprising polymer and the anionic polymer preferably 0.1-10 wt. %, more preferably 0.5-8 wt. %, based on the total weight of the hair cosmetic. Amounts of less than 0.1 wt. % result in an insufficient hair setting property, while those exceeding 10 wt. % increase stiffness, to thereby deteriorate the touch feeling.

Examples of nonionic polymers include polymers having as an essential component a repeating unit derived from an unsaturated monomer containing a pyrrolidone reing, amide group, N-alkyl-substituted amide group, polyether group, acetamide group or formamide group. Specific example of the polymer containing a pyrrolidone ring include polyvinyl pyrrolidone such as "Luviskol K-12, K-17, K-30, K-60, K-80 and K-90" (each, trade name; product of BASF AG) and "PVP K-15, K-30, K-60, K-90 and K-120" (each, trade name; product of ISP, Inc.); vinyl pyrrolidone/vinyl acetate copolymers such as "Luviskol VA28, VA37, VA55, VA64, and VA73" (each, trade name; product of BASF AG), "PVP/VA-735, PVP/VA-635, PVP/VA-535, PVP/VA-335, PVP/VA-235 and S-630" (each trade name; product of ISP, Inc.); and vinyl pyrrolidone/vinyl acetate/vinyl propionate copolymers such as "Ruviskol VAP343" (trade name; product of BASF AG).

Specific examples of the polymer containing as an essential component a repeating unit derived from an amide-, N-alkyl-substituted amide or polyether containig unsaturated monomer include radical homopolymers of an unsaturated monomer such as (meth)acrylamide, N-octyl (meth)acrylamide, hydroxyethyl (meth)acrylate, (meth)acrylic acid methoxypolyethylene glycol, (meth)acrylic acid methoxypolyethylene glycol polypropylene glycol; and radical copolymers with a C₁₋₂₄ alkyl(meth)acrylate, vinyl acetate or the like.

The weight ratio of the guanidino group-comprising polymer to the nonionic polymer preferably falls within a range from 1:10 to 10:1, more preferably from 1:5 to 10:1. When the ratio is less than 1:10, in the case of the hair cosmetics, the resulting cosmetic composition has insufficient flexibility, makes the hair sticky, has insufficient hair setting effects and causes problems such as flaking when combed or difficulty in setting the hair into a desired style due to static electricity. When the ratio exceeds 10:1, on the other hand, the resulting composition cannot impart sufficient hardness so that the hair thus set lacks tautness and has a heavy finish feeling, resulting in deterioration of the touch feeling thus obtained. The total amount of the guanidino group-comprising polymer and the nonionic polymer preferably 0.1-10 wt. %, more preferably 0.5-8 wt. %, based on the total weight of the hair cosmetic. Amounts of less than 0.1 wt. % provide an insufficient hair setting property, while those exceeding 10 wt. % increase stiffness and deteriorate the touch feeling.

Amphoteric polymers include polymers each containing as an essential component a repeating unit derived from an unsaturated monomer which contains a betaine group such as a carboxybetaine group, sulfobetaine group or phosphobetaine group; or polymers each containing as essential components both a repeating unit derived from an unsaturated monomer which contains an anionic group such as a carboxyl group, sulfonic acid group or phosphoric acid group and a repeating unit derived from an unsaturated monomer which contains a quaternary ammonium salt or a tertiary amino group. Specific examples of the polymer containing as an essential component a repeating unit derived from a betaine group containing unsaturated monomer include methacrylic carboxybetaine polymers, which are demethylaminoethyl methacrylate/alkyl methacrylate copolymers modified with a monohaloacetate, for example. "Yulaformer 205S, SM, AMPHOSET, 301, R102, R402, 510, 201, W (each, trade name; product of Mitsubishi Chemical). This technology is disclosed in JP-A-51-9732, JP-A-55-104209. JP-A-61-258804 and JP-7-285832.

Examples of the polymer which comprises as essential components both a repeating unit derived from an unsaturated monomer containing an anionic group such as carboxyl, sulfonic acid or phosphoric acid group and a repeating unit derived from unsaturated monomer containing a quaternary ammonium salt or a tertiary amino group include hydroxypropyl acrylate/butylaminoethyl methacrylate/octylamide acrylate copolymers such as "Amformer 28-4910, LV-71 and LV-47" (each, trade name; product of National Starch & Chemical Co.), which are polymers each comprising as essential components both a repeating unit derived from a carboxyl-containing unsaturated monomer and a repeating unit derived from a tertiary-amino-containing unsaturated monomer; and diallyldimethylammonium chloride/acrylic acid copolymers such as "Merquat 295" (tradename; product of Calgon), and diallyldimethylammonium chloride/acrylic acid/acrylamide copolymers such as "Merquat Plus 3330" (trade name; product of Calgon), which are polymers comprising as essential components both a repeating unit derived from a carboxyl-containing unsaturated monomer and a repeating unit derived from a quaternary-ammonium-salt-containing unsaturated monomer.

The weight ratio of the guanidino group-comprising polymer to the amphoteric polymer preferably falls within a range from 1:10 to 10:1, more preferably from 1:5 to 10:1. When the ratio is less than 1:10, in the case of the hair cosmetics, the resulting cosmetic composition has insufficient flexibility, makes the hair stiff, has insufficient hair setting effects to retain a desired style under high humidity conditions, and is not adequately removed by hair washing. When the ratio exceeds 10:1, on the other hand, the resulting composition does not impart sufficient hardness so that the hair thus set lacks tautness and has a heavy finish feeling, resulting in deterioration of the touch feeling thus obtained. In addition, upon combing after setting the hair with the composition, flaking occurs in which the film thus formed is divided into small flakes. The total amount of the guanidino group-comprising polymer and the amphoteric polymer preferably 0.1-10 wt. %, more preferably 0.5-8 wt. %, based on the total weight of the hair cosmetic. Amounts of less than 0.1 wt. % result in an insufficient hair setting property, while those exceeding 10 wt. % increase stiffness, to thereby deteriorate the touch feeling.

In addition to the guanidino group-comprising polymer and optionally contained another polymers, it is possible to incorporate into the hair, skin or nail cosmetics of the present invention the following components which are ordinarily used in cosmetics to the extent of not impairing the advantages of the present invention. Examples thereof include glycerides such as castor oil, cacao oil, mink oil, avocado oil, jojoba oil, macadamia nut oil and olive oil; waxes such as lanolin; esters such as isopropyl myrstate, octyldodecyl myristate, hexyl laurate and cetyl lactate; linear or branched higher alcohols such as cetyl alcohol, oleyl alcohol, stearyl alcohol, isostearyl alcohol, lauryl alcohol and 2-octyl dodecanol; linear or branched higher fatty acids such as lauric acid, stearic acid, myristic acid and oleyc acid; ethylene oxide and/or propylene oxide adducts of a higher alcohol such as polyoxyethylene lauryl ether, polyoxypropylene cetyl ether,and polyoxyethylene polyoxypropylene stearyl ether; amides such as oleic diethanolamide and lauric diethanolamide; cationic surfactans such as stearyl trimethylammonium chloride, distearyl dimethylammonium chloride and lauryl trimethylammonium chloride; anionic surfactants such as polyoxyethylene laurylether sulfate and polyoxyethylene lauryl sulfosuccinate salt; amphoteric surfactants such as imidazoline-type, alkylbetaine-type and aminoxide-type; protein derivatives and amino acid derivatives such as collagen hydrolyzates, keratin hydrolyzates and polyamino acid; vegetablr extracts, crude drugs, vitamins and ultraviolet absorbers such as oxybenzene, chelating agents such as EDTA-Na, antiseptics such as paraben, antioxidants, colorants, pigments and perfumes.

Embodiments of the hair, skin, and nail cosmetics of the present invention are described below.

### (Hair Cosmetics)

The hair cosmetic of the present invention comprises the cosmetic-use polymer of the present invention and can be in the form of a shampoo, rinse, treatment, set lotion, permanent wave solution, or the like. Since the cosmetic-use polymer of the present invention is particularly good as a hair set enhancer, hair cosmetics such as set lotions comprising the cosmetic-use polymer of the present invention as a hair set enhancer are preferred. Further, shampoos, rinses, treatments, permanent wave solutions, and the like containing the cosmetic-use polymer of the present invention as a conditioner are also covered under the hair cosmetic of the present invention.

The hair cosmetic of the present invention may be in the form of a liquid, cream, emulsion, gel, mousse, or the like. The hair cosmetic of the present invention may also comprise oils such as hydrocarbon oils, ester oils, and silicon oils; feel enhancers such as higher alcohols, polyethyleneglycol, and glycerin; sprays such as LPG and dimethylether; thickeners such as hydroxyethyl cellulose; and other hairstyling polymers such as polyvinyl pyrrolidone and amphoteric acrylic polymers to the extent that the effect of the present invention is not lost. Preservatives, ultraviolet absorbing agents, metal ion scavengers, antimildew agents, colorants, fragrance materials, foaming agents, foam stabilizers, and the like may also be suitably added. In addition, the cosmetic-use polymer of the present invention may be employed in combination with conventionally employed, known natural polymers and modified natural polymers.

The range of the content of the cosmetic-use polymer in the hair cosmetic of the present invention differs with the application and cannot be specified once and for all. However, a content of 0.1-10 weight percent is generally desirable. In the hair cosmetic, one or more types of guanidino group-comprising polymer may be employed.

Various other components contained along with the cosmetic-use polymer are described in detail below for individual applications of the hair cosmetic.

When the cosmetic-use polymer of the present invention is employed as a hair set enhancer, it can be employed as an additive in hair cosmetics of a variety of forms, such as aerosol hairsprays, pump hairsprays, foam hairsprays, hair mists, set lotions, hairstyling solutions, hair creams, and hair oils. All setting agents obtained by replacing some or all of the anionic, nonionic, and amphoteric polymers conventionally employed in hair fixatives with the cosmetic polymer of the present invention are covered under the present invention. To the extent that the effect of the present invention is not lost, known additives in the form of known polymers, fats and oils, moisturizers, solubilizing agents, emulsifiers, microbicides, and fragrance materials may be employed with the cosmetic-use polymer of the present invention.

Hair cosmetics for hair setting may be manufactured by dissolving, emulsifying, or dispersing the cosmetic-use polymer of the present invention and, as desired, the above-described additives, in a solvent. Solvents suitable for use are water, ethanol, isopropanol, other alcohols, and mixed solvents thereof. The cosmetic-use polymer of the present invention is desirably incorporated into hair cosmetics in a proportion of 0.1-10 weight percent. Further, solvents such as water and ethanol are desirably incorporated in a proportion of not less than 30 weight percent.

When formulated as a hair cosmetic (mousse) that can be sprayed out as a foam, the product desirably comprises 0.1-10 weight percent of the cosmetic-use polymer of the present invention, 0.1-5 weight percent of nonionic surfactants, 3-25 weight percent of liquified gas, and from 60 weight percent to the remainder of an aqueous solution comprised chiefly of water (at least 60 weight percent water is desirably contained in hair cosmetics). Examples of the nonionic surfactant are sorbitan fatty esters, glycerin fatty esters, polyoxyethylene sorbitan fatty esters, polyethylene glycol fatty esters, polyoxyethylene alkyl ethers, polyoxyethylene alkyl phenyl ethers, polyoxyethylene castor oil, and fatty acid alkanolamides.

When formulated as a hairspray, the product desirably comprises 0.1-10 weight percent of the cosmetic-use polymer of the present invention, 30-80 weight percent of a solvent, and 10-70 weight percent of a spray agent. Spray agents suitable for use are liquified gases and compressed gases. Examples are liquified gases such as liquified petroleum gas, dimethylether, and halogenated hydrocarbons, as well as compressed gases such as air, carbon dioxide gas, and nitrogen gas.

When the cosmetic-use polymer of the present invention is formulated as a conditioner, it can be employed as an additive in hair cosmetics in the form of shampoos, rinses, hair treatments, permanent solutions, and the like. Hair cosmetics such as shampoos can be prepared by dissolving, and/or dispersing the cosmetic-use polymer of the present invention along with other desired additives in a solvent. Examples of solvents suitable for use are water, ethanol, isopropanol, and other alcohols, as well as mixed solvents of the same. Further, when employed in hair treatments, in addition to the above-listed solvents, solvents in the form of hydrocarbons with boiling points of 50-300°C, or mixed solutions of the same with alcohols, may be employed.

Other components incorporated with the cosmetic-use polymer of the present invention are described in detail below for the various uses of hair cosmetics.

When the hair cosmetic of the present invention is formulated as a shampoo, known anionic, amphoteric, and nonionic surfactant base materials may be employed with the cosmetic-use polymer of the present invention. Known additives such as foaming agents, thickeners, hydrotropes, opacefiers, conditioning agents, bactericides, and fragrance materials may also be employed.

When the hair cosmetic of the present invention is formulated as a rinse, known cationic surfactants may be employed with the cosmetic-use polymer of the present invention. Known additives such as oils, cationic and amphoteric polymers, moisturizers, solubilizing agents, emulsifiers, thickeners, opacefiers, bactericides, hair-growth agents, and fragrance materials may also be employed.

When the hair cosmetic of the present invention is formulated as a permanent solution, known bromates, perborates, thioglycolic acids, salts thereof, cysteine, and other oxidizing and reducing agent base materials may be employed with the cosmetic-use polymer of the present invention. Known additives such as thickeners, stabilizers, opacefiers, conditioning agents, moisturizers, bactericides, and fragrance materials may also be employed.

When the hair cosmetic of the present invention is formulated as a hair treatment, known cationic surfactants and/or cationic polypeptides, cationic cellulose, cationic polysiloxane, and other cationic polymers may be employed with the cosmetic-use polymer of the present invention. Known additives such as oils, amphoteric polymers, moisturizers, solubilizing agents, emulsifiers, thickeners, opacefiers, bactericides, hair-growth agents, and fragrance materials may also be employed.

### (Skin and Nail Cosmetics)

When the present invention is in the form of a skin and nail cosmetic, there are no specific limitations. However, examples of skin cosmetics are: creams, emulsions, other basic cosmetics, foundations, toilet powders, blushes, eyeshadows, lipsticks, other makeup products, and antiperspirants. Examples of nail cosmetics are nail colors, nail care creams, nail enamel basecoats, and nail enamel overcoats.

Skin and nail cosmetics of the present invention contain the cosmetic-use polymer of the present invention. The content thereof is desirably 1-30 weight percent of the cosmetic. The cosmetic of the present invention can be manufactured by dissolving, emulsifying, or dispersing the formulation components, including the cosmetic polymer of the present invention, in water, an alcohol solvent such as ethanol, an ester solvent such as ethyl acetate, a ketone solvent such as methyl ethyl ketone, or a hydrocarbon such as liquid paraffin or Vaseline.

Further, the present invention provides a method of processing keratinous substances comprising the step of applying the guanidino group-comprising polymer of the present invention to keratinous substances. In this processing method, the amount of guanidino group-comprising polymer applied to the keratinous substance is an amount effective for treatment. One embodiment of implementing this processing method is a method of processing keratinous substances comprising the step of applying on keratinous substances a solution obtained by dissolving, emulsifying, and/or dispersing the guanidino group-comprising polymer in solvent. The application may take various forms, such as coating and spraying.

The keratinous substance is desirably skin, hair, and/or nails.

### Examples

The present invention will be specifically explained with reference to the following examples. The materials, regents, ratios, procedures and so forth shown in the following examples can be optionally changed so long as such change does not depart from the spirit of the present invention. Therefore, the scope of the present invention is not limited by the following examples.

### <Preparing Example 1 (Example of Preparing the Cosmetic-Use Polymer of the Present Invention)>

To a reactor equipped with reflux condenser, dropping funnel, temperature gauge, nitrogen gas inlet tube, and stirring device were charged 50 weight parts of glycidyl methacrylate, 30 weight parts of methyl methacrylate, 10 weight parts of butyl methacrylate, 10 weight parts of stearyl methacrylate, and 150 weight parts of anhydrous ethanol. Subsequently, 0.6 weight part of 2,2'-azobisisobutyronitrile was added. The reactor was backfilled with nitrogen, heated from room temperature to 80°C over 40 min, and reacted for 8 hrs at 80°C. It was then cooled to 60°C, yielding a polymerization reaction solution.

Next, aminoguanidino in an equimolar to the glycidyl methacrylate was added dropwise over 1 hr to the polymerization reaction solution and stirring was continued for another 20 hrs, yielding guanidino group-comprising polymer. The end of the reaction was confirmed by measurement of the epoxy value of the reaction solution. The polymer obtained was labeled "P-1."

The weight average molecular weight of the polymer obtained was 1.1·10⁵.

### <Preparing Example 2 (Example of Preparing the Cosmetic-Use Polymer of the Present Invention)>

To a reactor identical to that employed in Preparing Example 1 were charged 40 weight parts of glycidyl methacrylate, 10 weight parts of methoxydiethyleneglycol methacrylate, aminoguanidine in an equimolar to the glycidyl methacrylate, and 150 weight parts of anhydrous ethanol. The mixture was reacted for 8 hrs at 80°C and then cooled to 40°C. The end of the reaction was confirmed by measurement of the epoxy value. Subsequently, to the reactor were charged 30 weight parts of methyl methacrylate, 10 weight parts of butyl methacrylate, and 10 weight parts of stearyl methacrylate. A further 0.6 weight part of 2,2'-azobisisobutyronitrile was then added. After backfilling with nitrogen, the reactor was heated from room temperature to 80°C over 40 min, reacted for 8 hrs at 80°C, and cooled to 60°C, yielding guanidino group-comprising polymer. The polymer obtained was labeled "P-2."

The weight average molecular weight of the polymer obtained was 1.0·10⁵.

### <Preparing Example 3 (Example of Preparing Comparative Polymer)>

To a reactor identical to that employed in Preparing Example 1 were charged 50 weight parts of N,N-dimethylaminoethyl methacrylate, 30 weight parts of methyl methacrylate, 10 weight parts of butyl methacrylate, 10 weight parts of stearyl methacrylate, and 150 weight parts of anhydrous ethanol. To the reactor was also charged 0.6 weight part of 2,2'-azobisisobutyronitrile. Under a nitrogen atmosphere, the mixture was reacted for 8 hrs at 80°C and then cooled to 60°C. Next, hydrogen peroxide (as a 31 weight percent aqueous solution) in an equimolar to the N,N-dimethylaminoethyl methacrylate was added dropwise over 1 hr and stirring was continued for another 20 hrs to oxidize the dimethylamino group. The end of the oxidation reaction was confirmed by measuring the amine value of the reaction solution. The polymer obtained was labeled "P-3."

The weight average molecular weight of the polymer obtained was 1.1·10⁵. N-O absorption was observed in the infrared absorption spectrum, confirming the generation of amineoxide groups.

### <Preparing Example 4 (Example of Preparing Comparative Polymer)>

To a reactor identical to that employed in Preparing Example 1 were charged 50 weight parts of N,N-dimethylaminoethyl methacrylate, 30 weight parts of methyl methacrylate, 10 weight parts of butyl methacrylate, 10 weight parts of stearyl methacrylate, and 150 weight parts of anhydrous ethanol. To the reactor was also charged 0.6 weight part of 2,2'-azobisisobutyronitrile. Under a nitrogen atmosphere, the mixture was reacted for 8 hrs at 80°C and then cooled to 60°C. Next, diethyl sulfate in an equimolar to the N,N-dimethylaminoethyl methacrylate was added dropwise over 1 hr and stirring was continued for another 20 hrs conduct ethyl cationization of the dimethylamino groups. The polymer obtained was labeled "P-4."

The weight average molecular weight of the polymer obtained was 1.1·10⁵.

### <Preparing Example 5 (Example of Preparing the Cosmetic-Use Polymer of the Present Invention)>

Polymerization and introduction of a guanidino group were conducted in the same manner as in Preparing Example 1 to obtain a polymer, with the exception that in Preparing Example 1, the composition of the monomers charged to the reactor was 20 weight parts of glycidyl methacrylate, 30 weight parts of methyl methacrylate, 20 weight parts of butyl methacrylate, and 30 weight parts of stearyl methacrylate. The polymer obtained was labeled "P-5."

The weight average molecular weight of the polymer obtained was 1.0·10⁵.

The structural formulas of polymers P-1 through P-4 obtained above are given below. Only the repeating units are indicated in the structural formulas. The structure of P-5 is identical to that of P-1 with the exception that the ratio of repeating units is different.
P-1 : A mixture of (P-1a) and (P-1b) 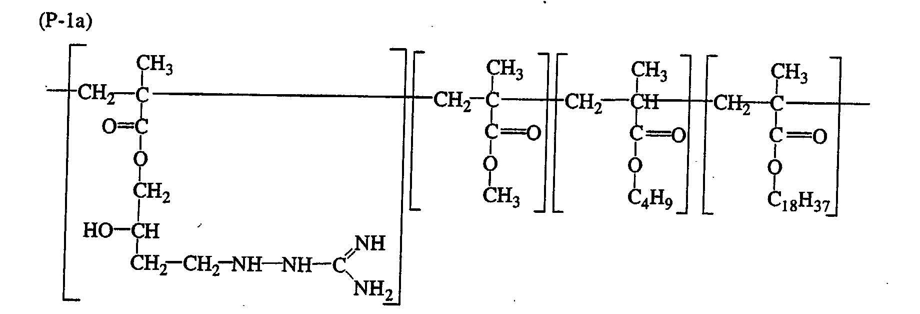 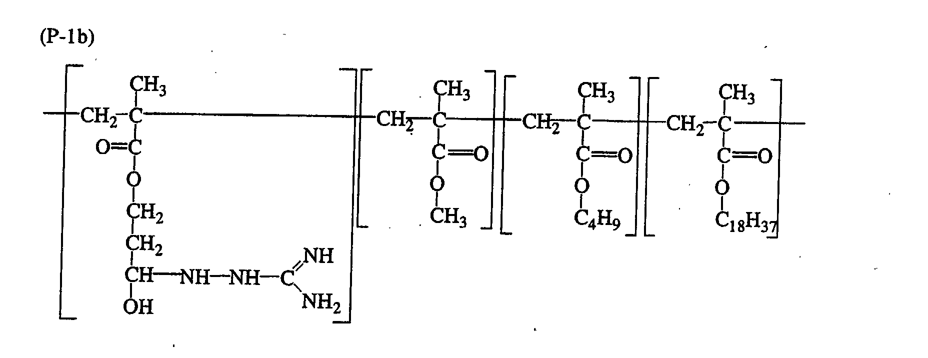 P-2 : A mixture of (P-2a) and (P-2b)

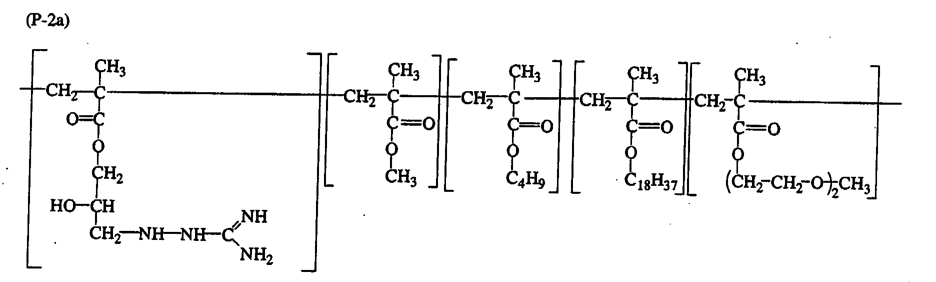

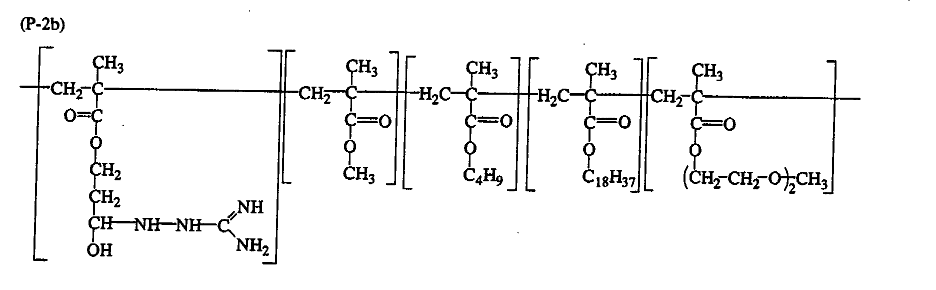

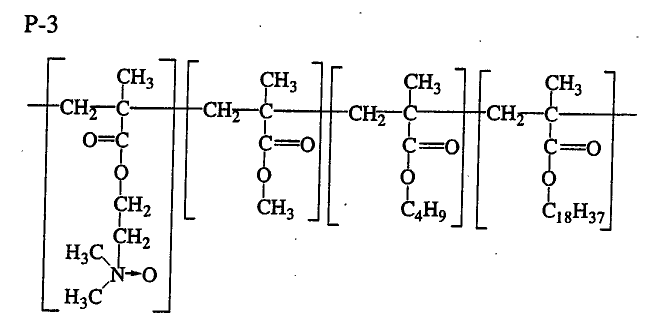

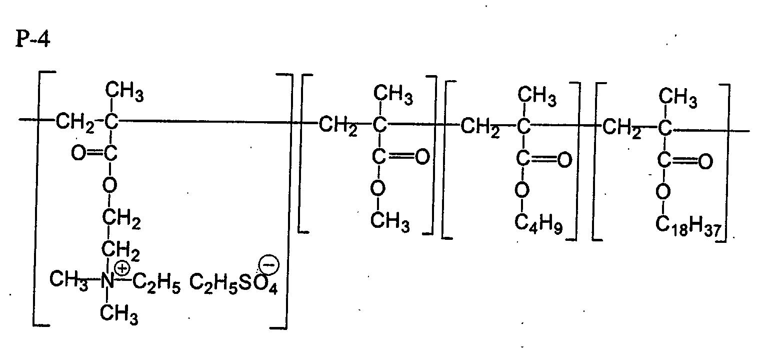

### <Water Solubility Evaluation>

One weight part each of the polymers of P-1 through P-4 was heated and stirred for 2 hrs at 60°C with 99 weight parts of deionized water, cooled, and left standing for one day. The transmittance at a wavelength of 655 nm of the aqueous solutions after standing was measured and water solubility was evaluated according to the following criteria. The results are given in Table 1.
- ○:: Transmittance of 70-100 percent.
- Δ:: Transmittance of 30-70 percent.
- □:: Transmittance of 0-30 percent.

**Table 1.**

| Polymer | Water Solubility |
|---|---|
| P-1 | ○ |
| P-2 | ○ |
| P-3 | ○ |
| P-4 | ○ |

### (Example 1 :Shampoo)

Shampoo 1 was prepared with the following composition.

| | (weight percent) |
|---|---|
| Polyoxyethylene sodium lauryl sulfate (3 EO adduct) | 16% |
| Lauroyl diethanolamide | 2% |
| "P-1" | 1.5% |
| Fragrance materials | 0.2% |
| Preservative | 0.1% |
| Pigments | trace amount |
| Pure water | to make 100% |

When Shampoo 1 was employed, it imparted a smooth feel to the hair following washing and the hair was easy to comb. Further, after drying, the hair had good luster and shine, as well as a smooth feel. Further, a comb passed readily through the hair after drying. Still further, when the hair was washed repeatedly with this shampoo, no tackiness or undesired effects developed.

Shampoo 2 was prepared with the same composition as Shampoo 1 with the exception that polymer P-2 was employed in place of polymer P-1. When the same evaluation was conducted, the same results were obtained as for Shampoo 1.

Shampoo 3 was prepared with the same composition as Shampoo 1 with the exception that polymer P-3 was employed in place of polymer P-1. When the same evaluation was conducted, in contrast to Shampoos 1 and 2, smoothness during washing was inadequate.

Shampoo 4 was prepared with the same composition as Shampoo 1 with the exception that polymer P-4 was employed in place of polymer P-1. When the same evaluation was conducted, in contrast to Shampoos 1 and 2, the hair became tacky following drying.

### (Example 2 :Spray-Type Setting Agent)

A diluted original solution of the following composition was loaded into a spray can and the can was packed with liquified petroleum gas to prepare Hairspray 2.

| Diluted original solution | (weight percent) |
|---|---|
| "P-2" | 4% |
| Anhydrous ethanol | 46% |
| Liquified petroleum gas (3 Kg/cm²G, 20°C) | 50% |

When the Hairspray was sprayed onto the hair, good setting strength was imparted to the hair, the hair had good gloss and shine, and a smooth feel was imparted. Further, when Hairspray 2 was repeatedly applied and the hair was repeatedly washed, no undesirable effect such as tackiness or an unnatural feel due to accumulation developed.

Hairspray 1 was prepared with the same composition as Hairspray 2 with the exception that polymer P-1 was employed in place of polymer P-2. When the same evaluation was conducted, the same results were achieved as for Hairspray 2.

Hairspray 3 was prepared with the same composition as Hairspray 2 with the exception that polymer P-3 was employed in place of polymer P-2. When the same evaluation was conducted, in contrast to when Hairsprays 2 and 1 were employed, no smooth feel was imparted to the hair following application.

Hairspray 4 was prepared with the same composition as Hairspray 2 with the exception that polymer P-4 was employed in place of polymer P-2. When the same evaluation was conducted, in contrast to when Hairsprays 2 and 1 were employed, the setting strength of the hair was weak and a tacky feel was imparted to the hair following application.

### (Example 3 : Foam-Type Setting Agent)

Foam-Type Setting Agent 1 was prepared with the following composition.

| Diluted original solution | (weight percent) |
|---|---|
| "P-1" | 2% |
| Yuka Foamer AM-75R205S (described in detail below) | 2% |
| Polyoxyethylenecetylether (10 EO adduct) | 0.3% |
| Polyoxyethylenecetylether (2 EO adduct) | 0.1% |
| Pure water | 83.6% |
| Liquified petroleum gas (3 Kg/cm²G, 20°C) | 12% |

"Yuka Foamer AM-75205S" is a carboxybetaine amphoteric polymer sold by Mitsubishi Chemicals (Ltd.).

Foam-Type Setting Agents 2-4 were prepared with the same composition as Foam-Type Setting Agent 1 with the exception that polymers P-2 through P-4 were employed in place of polymer P-1. The Foam-Type Setting Agents 1-4 obtained were applied to hair and the following evaluation was conducted. The evaluation results are given in Table 2 below.

### (1) Setting Strength Evaluation

Tresses of hair 23 cm in length and 2.0 g in weight were immersed in each of the samples, removed, lightly constricted, wound on rods 1 cm in diameter, and dried. Subsequently, they were removed from the rods, yielding curls. The tresses of hair were vertically hung for 30 min in a thermo-hygrostat that had been preadjusted over 3 hrsto a temperature of 30°C and a humidity of 90 percent, and the setting strength was evaluated 30 min later based on the extension ratio of the hairs. Specifically, the curling strength prior to placement in the thermo-hydrostat was taken as 100 percent, the curl retention rate after extension of the hair tresses was calculated, and the setting strength was evaluated based on the following criteria.
- ○:: Retention rate of not less than 80 percent; almost no change.
- Δ:: Retention rate of 60-80 percent; some extension.
- X:: Retention rate of less than 60 percent; marked extension observed.

### (2) Evaluation of Hair Tackiness

Tresses of hair 15 cm in length and 10 g in weight were coated with individual samples of about 2 g and left standing at room temperature following the coating. The tackiness after 30 min was then evaluated based on the following criteria by a ten-person panel.
- ⓞ:: No tackiness noted by any member of ten-member panel.
- ○:: Tackiness noted by 1-3 members of ten-member panel.
- Δ:: Tackiness noted by 4-7 members of ten-member panel.
- X:: Tackiness noted by 8 or more members of ten-member panel.

### (3) Flexibility of hair tresses Evaluation

Tresses of hair 15 cm in length were coated with 1.5 g of individual samples, immediately arranged to a width of 2 cm, dried, and placed for 1 hr in a thermo-hygrostat at a temperature of 23°C and a relative humidity of 60 percent. They were then placed on a support base at a spacing of 65 mm, the maximum load when bent at a fixed rate in the center was measured, and flexibility of the hair tresses was evaluated based on the following criteria.
- ○:: Maximum load less than 100 g, no unnatural feel of polymer, good flexibility.
- Δ:: Maximum load not less than 100 g but less than 150 g, flexibility present, but unnatural polymer feel remained.
- X:: Maximum load exceeded 150 g, unnatural polymer feel present, poor flexibility.

**Table 2**

| | Polymer | Setting Strength (Retention Rate %) | Hair Tackiness | Flexibility (Maximum Load g) |
|---|---|---|---|---|
| Example 3 | P-1 | ○ | ⓞ | ○ |
| | | (85) | | (60) |
| | P-2 | ○ | ⓞ | ○ |
| | | (87) | | (65) |
| | P-3 | ○ | ○ | ○ |
| | | (95) | | (95) |
| | P-4 | × | Δ | ○ |
| | | (55) | | (70) |

The results in Table 2 reveal that foam-type setting agents employing P-1 and P-2, cosmetic-use polymers of the present invention, imparted good setting strength and flexibility to the hair and afforded good use feeling without imparting tackiness to the hair. By contrast to when P-1 and P-2 were employed, the setting agent employing Comparative Polymer P-3 imparted a certain feeling of tackiness to the hair and did not impart a smooth feel. The setting agent employing Comparative Polymer P-4 afforded poorer hair setting strength than polymers P-1 and P-2, and imparted a feeling of tackiness to the hair.

### (Example 4 : Lotion Setting Agent)

Setting Lotion 2 was prepared with the following composition.

| | (weight percent) |
|---|---|
| "P-2" | 3% |
| Pure water | 60% |
| Anhydrous ethanol | 37% |

When the Setting Lotion was applied to the hair and evaluated in the same manner as in Example 3, the same good results were obtained as in Example 3.

Setting Lotion 1 was prepared with the same composition as Setting Lotion 2 with the exception that polymer P-1 was employed in place of polymer P-2. When the same evaluation as in Example 3 was conducted, the same good results as in Example 3 were obtained.

Setting Lotion 3 was prepared with the same composition as Setting Lotion 2 with the exception that polymer P-3 was employed in place of polymer P-2. When the same evaluation as in Example 3 was conducted, the results were inferior to those obtained when P-1 and P-2 were employed in that a certain tackiness was imparted to the hair and a smooth feel was not imparted.

Setting Lotion 4 was prepared with the same composition as Setting Lotion 2 with the exception that polymer P-4 was employed in place of polymer P-2. When the same evaluation as in Example 3 was conducted, the hair setting strength was weaker than when P-1 and P-2 were employed and a tackiness was imparted to the hair.

### (Example 5 : Skin Cosmetic)

Skin Cosmetic 1 was prepared with the formulation indicated in Table 3 below.

**Table 3.**

| | | Skin Cosmetic 1 | Skin Cosmetic 2 | Skin Cosmetic 3 |
|---|---|---|---|---|
| Polymer | "P-5"(30% solution) | 15.0% | - | - |
| | Cationic Polymer "JR-400" (30% solution) | - | 15.0% | - |
| | Anionic Polymer "ES225" (30% solution) | - | - | 15.0% |
| Polyether Modified Silicone "SH3771C" (manufactured by Toray Dow Corning Silicone(ltd.)) | | 0.3% | 0.3% | 0.3% |
| Water | | 84.7% | 84.7% | 84.7% |
| In above table, "%" means "wt. %". | | | | |

When the Skin Cosmetic 1 was applied to the skin, there was no unnatural feel or tackiness following drying, and a smooth film was obtained that produced a pleasant feeling.

Skin Cosmetic 2 was prepared in the same manner as in Example 5 with the exception that in the formulation in Table 3, a 30 percent ethanol solution of cationic polymer ("JR-400" from Union Carbide) was employed in place of polymer P-5. When Skin Cosmetic 2 was applied to the skin, there was considerable tackiness and a smooth feel was not imparted.

Skin Cosmetic 3 was prepared in the same manner as in Example 5 with the exception that in the formulation in Table 3, a 30 percent ethanol solution of anionic polymer ("Gantretz ES 225" from ISP Co.) was employed in place of polymer P-5. When Skin Cosmetic 3 was applied to the skin, there was considerable tackiness, a smooth feel was not imparted, and separation occurred with repeated rubbing.

### (Example 6 : Nail Cosmetic)

Nail Cosmetic 1 was prepared by dispersing and mixing the components of the following formulation in a bead mill for 1 hr.

| | (weight percent) |
|---|---|
| Polymer P-5 | 50.0% |
| | (as solid portion 15.0%) |
| Isopropanol | 10.0% |
| Red No. 226 | 0.1% |
| Titanium oxide | 4.9% |
| Ethanol | 30.0% |
| | (as total ethanol quantity 65.0%) |
| Bentonite | 5.0% |
| ("Benton EW" from National Reed Co.) | |

Nail Cosmetic 2 was prepared in the same manner as Nail Cosmetic 1 with the exception that a cationic polymer ("JR-400" from Union Carbide) was employed in place of polymer P-5 in a quantity yielding the same solid portion.

Nail Cosmetic 3 was prepared in the same manner as Nail Cosmetic 1 with the exception that an anionic polymer ("Gantretz ES 225" from ISP Co.) was employed in place of polymer P-5 in a quantity yielding the same solid portion.

The Nail Cosmetics 1-3 obtained were subjected to evaluations (1)-(5) below. The evaluation results are given in Table 4.

### (1) Pencil Hardness

The test was conducted in accordance with 6.14 of JIS K5400 with the exception that a glass sheet was employed in place of a steel plate as the coated surface.

### (2) Flexibility of film Test

Each of the samples was tested in accordance with 6.16 of JIS K5400 and evaluated according to the following criteria.
- ○:: Determined to resist bending.
- X:: Determined not to resist bending.

### (3) Water Repellency

Each of the samples was applied with a bar coater to a thickness of 0.1 mm on a glass sheet, dried, and immersed for 1 hr in pure water. Subsequently, the samples were evaluated for film peeling and discoloration such as clouding based on the following criteria.
- ○:: No separation, no discoloration.
- Δ:: No separation, but discoloration (clouding) present.
- X:: Both separation and discoloration present.

### (4) Drying Property

Each of the samples was applied with a bar coater to a thickness of 0.1 mm on a glass sheet and the time until there was no change when touched with a finger was measured.
- ○:: Less than 10 minutes.
- X:: 10 minutes or more.

### (5) Adhesion

Under conditions of 25°C and 60 percent relative humidity, each sample was applied to a nail with a nail enamel brush and dried. Twenty minutes later, the film was scraped off the surface with a microspatula, the degree of scraping (difficulty of scraping) was observed, and an evaluation was made based on the following criteria.
- ○:: Good: scraping was difficult.
- X:: Poor: scraping was easy.

**Table 4.**

| | Nail Cosmetic 1 | Nail Cosmetic 2 | Nail Cosmetic 3 |
|---|---|---|---|
| (1) Pencil Hardness | HB | 6B | H |
| (2) Flexibility of film Test | ○ | ○ | × |
| (3) Water Repellency | ○ | × | × |
| (4) Drying Property | ○ | × | ○ |
| (5) Adhesion | ○ | ○ | × |

The present invention, as described above, provides a cosmetic-use polymer, and a method of preparing the same, with good adhesion and film-forming properties on skin, nails, and hair, high solubility in water, and a good use feeling without imparting a feeling of tackiness or unnaturalness due to film formation. Further, the present invention provides a cosmetic with good adhesion and film-forming properties on skin, nails, and hair and a good use feeling without imparting a feeling of tackiness or unnaturalness due to film formation. Still further, the present invention provides a hair cosmetic with good adhesion and film-forming properties on the hair and a good use feeling without imparting a feeling of tackiness or unnaturalness due to film formation. Still further, the present invention provides a hair cosmetic that is readily removed by rinsing with water and affords good setting strength and flexibility.

## Claims

1. A cosmetic-use polymer having a weight average molecular weight of 5.0·10³-1.0·10⁷ and comprising a repeating unit having at least the guanidino group denoted by general formula (I) below 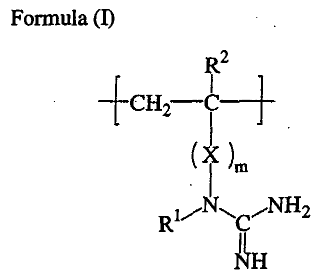 (where, in the formula, R¹ denotes a hydrogen atom or hydrocarbon group, R² denotes a hydrogen atom or methyl group, X denotes a bivalent connecting group, m denotes 0 or 1, and the guanidino group in the formula is optionally a salt to which an acid has been added).

2. The cosmetic-use polymer of Claim 1, wherein in general formula (I), X denotes a connecting group comprising one or more members selected from the group of bivalent groups listed below 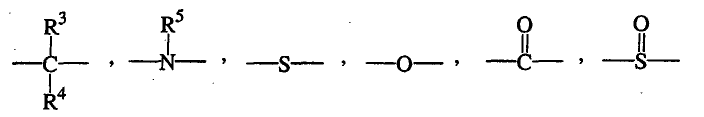 (where, in the formula, R³, R⁴, and R⁵ each independently denote a hydrogen atom, alkyl group with 1-24 carbon atoms, aryl group, arylalkyl group, or hydroxy group).

3. The cosmetic-use polymer of Claim 1 or 2, wherein said guanidino group-comprising repeating unit is denoted by general formula (II) below 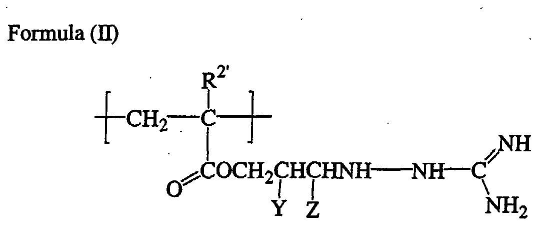 (where, in the formula, R^{2'} denotes a hydrogen atom or methyl group, one from among Y and Z denotes a hydrogen atom and the other denotes a hydroxy group, and the guanidino group in the formula is optionally a salt to which an acid has been added).

4. The cosmetic-use polymer of any one of Claims 1-3 comprising not less than 5 weight percent of said guanidino group-comprising (including a salt with an added acid) repeating unit.

5. The cosmetic-use polymer of any one of Claims 1-3 comprising not less than 15 weight percent of said guanidino group-comprising repeating unit.

6. The cosmetic-use polymer of any one of Claims 1-5 further comprising a repeating unit derived from the compound denoted by general formula (V) below Formula (V) 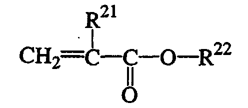 (where, in formula (V), R²¹ denotes a hydrogen atom or methyl group and R²² denotes an alkyl group with 1-24 carbon atoms).

7. A cosmetic comprising a polymer of any one of Claims 1-6.

8. The cosmetic of Claim 7 in the form of a hair cosmetic.

9. The cosmetic of Claim 7 in the form of a skin cosmetic.

10. The cosmetic of Claim 7 in the form of a nail cosmetic.

11. The cosmetic of Claim 8, wherein said hair cosmetic is a hair fixative.

12. The cosmetic of any one of Claims 7-11 comprising at least one member selected from the group consisting of water, alcohol solvents, ester solvents, ketone solvents, and hydrocarbon solvents.

13. The cosmetic of any one of Claims 7-11 comprising at least one member selected from the group consisting of water and alcohol solvents.

14. A method of using as a cosmetic a guanidino group-comprising polymer having a weight average molecular weight of 5.0·10³-1.0·10⁷ and having a repeating unit comprising at least the guanidino group denoted by general formula (I) bellow; 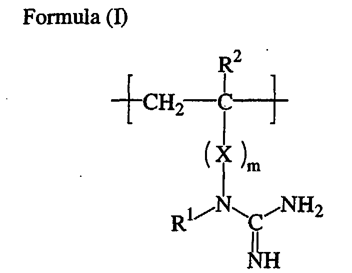 (where, in the formula, R¹ denotes a hydrogen atom or hydrocarbon group, R² denotes a hydrogen atom or methyl group, X denotes a bivalent connecting group, m denotes 0 or 1, and the guanidino group in the formula is optionally a salt to which an acid has been added).

15. The method of using of Claim 14, wherein said keratinous substance is hair, skin, or nails.

16. A method of preparing guanidino group-comprising polymer of Claim 1 comprising the step of preparing a monomer having at least a guanidino group (including a salt with an added acid) and the step of polymerizing said monomer alone or with another monomer.

17. A method of preparing guanidino group-comprising polymer of Claim 1 comprising the step of polymerizing a nitrogen-comprising monomer alone or with another monomers to obtain a nitrogen-comprising polymer and the step of guanidinizing said nitrogen-comprising polymer.

18. A method of preparing guanidino group-comprising polymer of Claim 1 comprising the step of polymerizing a monomer having a reactive functional group alone or with another monomers to obtain a polymer having a reactive functional group and the step of reacting said polymer with a compound having both a guanidino group and a reactive group capable of reacting with said functional group.
